Europäisches Patentamt

European Patent Office

Office européen des brevets

(11) Publication number: **0 274 856 B1**

## EUROPEAN PATENT SPECIFICATION

(45) Date of publication of patent specification: **03.06.92**  (51) Int. Cl.⁵: **A62D 3/00**, C12N 1/36, C02F 3/12

(21) Application number: **87310689.2**

(22) Date of filing: **04.12.87**

(54) **Microbial degradation of waste.**

(30) Priority: **08.12.86 GB 8629301**

(43) Date of publication of application:
**20.07.88 Bulletin 88/29**

(45) Publication of the grant of the patent:
**03.06.92 Bulletin 92/23**

(84) Designated Contracting States:
**AT BE CH DE ES FR GB GR IT LI LU NL SE**

(56) References cited:
**EP-A- 0 075 885
EP-A- 0 095 049
GB-A- 2 010 327
US-A- 3 756 947
US-A- 3 940 332**

(73) Proprietor: **THE UNIVERSITY OF KENT AT CANTERBURY**

**Kent, Canterbury CT2 7NJ(GB)**

(72) Inventor: **Knowles, Christopher John**
**5 Harbledown Park**
**Canterbury Kent CT2 8NR(GB)**
Inventor: **Wyatt, Jeremy Michael**
**3 Kestrel Avenue**
**Herne Hill London SE24 0ED(GB)**

(74) Representative: **Froud, Clive et al**
**Elkington and Fife Prospect House 8 Pembroke Road**
**Sevenoaks, Kent TN13 1XR(GB)**

EP 0 274 856 B1

**Description**

This invention relates to the microbial degradation of waste; more particularly, it relates to a biodegradation technique which may be applied to various toxic waste effluents which cannot be readily biodegraded using adapted activated sludge technology

The growth of particularly the chemical industry this century has generated a wide variety of man-made (anthropogenic) compounds. Such compounds may cause problems of disposal and, if they enter the environment, could have a detrimental effect.

The production of specific chemicals is generally associated with the formation of large amounts of undesired by-products and wastes. Microbial degradation of such wastes is rapidly being adopted as a convenient, cost-effective method of removing potential pollutants. The efficiency of such microbial processes depends on the susceptibility of the compounds in the waste effluent to microbial attack. Organic compounds are often classified as "biodegradable", "persistent" or "recalcitrant". Biodegradation is the biological transformation of an organic chemical to another form; the extent of the transformation is not implied. However, this term usually infers that the chemical may be mineralised. The term recalcitrance has been defined as the inherent resistance of a chemical to any degree of biodegradation, while persistence infers that it fails to undergo biodegradation under a specified set of conditions.

Fortunately, many chemicals used in industry today are also naturally occurring (biogenic), thereby being biodegradable. Many others, however, have novel chemical structures to which microorganisms have not been previously exposed, i.e. they are xenobiotic. Interestingly, many of these are also biodegradable. Continuous culture techniques have been used to select bacteria capable of degrading xenobiotics previously thought to be non-degradable, e.g. the azo dyes and the pesticide "Dalapon".

In pure culture, the adaptation of a microorganism to degrade a novel substrate, for example a xenobiotic, requires extensive mutation and the fortuitous acquisition of a novel metabolic pathway or genetic recombination with another organism to gain the necessary enzyme(s) for degradation of the xenobiotic. However, with a mixed culture the organisms may act in concert, combining the catabolic potential thereof to complete the mineralisation of compounds which the individual community members are unable to achieve. For example, the degradation of xenobiotics, such as alkylbenzene detergents and pesticides, has been achieved using mixed microbial populations. Continuous enrichment has been used as a method to isolate competitive microbial communities, while classical enrichment generally leads to isolation of in dividual microorganisms.

The regulation of mixed substrate utilization by a pure culture has been described. Mixed substrate utilization by mixed cultures is, however, more complex and the individual metabolic processes occurring cannot be easily studied.

Cometabolism is often required for the degradation of xenobiotics. Cometabolism has recently been redefined as the transformation of a non-growth substrate in the obligate presence of a growth substrate or another transformable compound. There are many organic compounds, such as chlorinated pesticides, that are biologically modified in the environment, but individual organisms that may use such compounds as carbon or energy sources have been difficult to isolate. One organism modifies the substrate such that second and subsequent organisms may use the product as a substrate to effect further transformations.

The chemical composition of industrial wastewaters is often complex, involving a large number of compounds. However, compared to domestic and municipal sewage where the number of pollutant compounds runs into hundreds if not thousands, industrial wastewater frequently contains less than 100 pollutant compounds, with more than 80 per cent of the pollutant load being represented by less than 10 key compounds. The strategies that are generally applied for disposal of industrial wastes are as follows: improvement and modification of process, for example reduction of the quantities of wastes by recycling; chemical hydrolysis; thermal oxidation (incineration); wet air oxidation; microbial oxidation or biodegradation; activated sludge systems; biodegradation with specialized microorganisms in pure or mixed cultures.

Biotreatment systems have developed along two maj or strategies, firstly the adaptation of classical activated sludge systems and other effluent treatment systems to biodegrade the waste water, secondly and more recently the use of specialised microbial cultures of microorganisms with unique metabolic capabilities.

(For example: EP-A-95049 relates to a microbial culture system comprising activated sludge and a supplemental microbial inoculant acclimated to metabolize chemical wastewaters and chemical waste landfill leachates containing recalcitrant halogenated organics; EP-A-75885 concerns certain microorganisms of the genera Pseudomonas which are capable of metabolizing chlorinated organic compounds to carbon dioxide, water and salt; US-A-3,940,332 relates to a process for the microbiological degradation of nitriles and cyanides contained in a waste water effluent using microorganisms of a particular Nocardia strain; US-A-3,756,947 proposes the addition to sludge of microorganisms selected from the genera Alcaligenes and Achromobacter for a similar purpose; and GB-A-2,010,327 relates to storage-stable preparations of microorganisms for use in the degradation of products of industrial organic syntheses. While these references may be regarded as examples of the closest prior art, they are clearly well-removed from the present invention.)

The present invention relates to a technique of the latter type to obtain an adapted microbial population, which may be used in an activated sludge system or in other effluent treatment systems, which population could not be obtained by classical techniques.

The present invention provides a process for the microbial degradation of waste characterised in that it comprises determining the significant constituent(s) of a waste, providing one or more microorganisms able at least partially to degrade and/or biotransform and/or mineralise each determined constituent of the waste,, optionally provided one or more other microorganisms capable of furthering mineralisation of biotransformation or partial degradation product (s), growing one or more mixed cultures of at least some of the microorganisms on a synthetic mixture of at least some of the determined constituents of the waste and utilising the adapted population of microorganisms substantially to mineralise the actual waste.

Generally, the mixed cultures are further adapted with a view to optimising mineralisation of the synthetic waste. This may involve sequential adaptation by addition of one or more of the determined constituents and/or by dilution and/or by switching between batch and continuous culture. Also, the adapted population may be optimised substantially to mineralise the actual waste.

Inasmuch as the concept of the present invention is generally applicable, the selection of microorganisms in a given instance is of secondary importance. Generally, the microorganisms are identified and isolated in conventional manner.

Although of general applicability, the present process will be illustrated with particular regard to the microbial degradation of the waste effluents produced during the commercial manufacture of acrylonitrile (AN). It will be appreciated that the present invention may be applied to various toxic waste effluents which cannot be readily biodegraded using adapted activated sludge technology.

The photographic processing industry produces a wide variation in the size and nature of its effluents, some having BOD concentrations as high as 300 mg/litre. Typical constituents include acetate, benzyl alcohol, citrate, ethylene diamine, ethylene glycol, 2-methyl-1,4-phenylene diamine, ferrocyanide, formalin, hydroquinone, thiocyanate and thiosulphate. In pharmaceutical manufacture, effluents may be produced from separation and purification operations. Two typical wastes are mixed non-halogenated solvents containing, for example, acetone, butyl acetate, dimethylformamide, water, ethanol, toluene, benzene and isopropanol, and mixed waste solvents, such as pyridine, benzene, toluene, ethylene dichloride, isobutanol and methanol. Biocide manufacture may produce aqueous streams containing low concentrations of brominated and chlorinated hydrocarbons, such as dichloroethane. Metal finishing wastes may include sodium carbonate, metasilicates, borates, chromates, cyanide, soaps, surfactants, sequestering agents, rust inhibitors, tartrates, ammonium salts and antitarnishing agents. The present technique may also be applied to such situations, for example.

In general terms, a given industrial waste is first analysed by a variety of conventional techniques, such as HPLC, GLC and GC-MS, in order to determine the major individual components and the concentrations thereof.

Microorganisms capable of biodegrading each individual component are then isolated by classical enrichment techniques, the component of the effluent being utilized as for example source of carbon or nitrogen or both carbon and nitrogen. If there are components in the waste that fail to support microbial growth, for example because of the toxicity thereof, enrichment may be performed using one or more analogues or related metabolites or metabolic precursors of the component. An analogue is selected such that its metabolism is related to or via the catabolism of particular molecular constituents also found in the waste component. When microbial isolates are obtained using analogues, the ability thereof to biotransform the waste component should be tested.

By establishing a conventional batch or continuous culture using one or more of the components of the effluent which are readily biodegraded by the resulting microorganisms, a stable degradative population may be obtained, the microbial inocula being a defined mixture of all or some of the isolates obtained above. If a low concentration of a particular toxic component is added in continuous culture to the media feed, the culture may be adapted to biodegrade this component as well as the other constituents by two methods, firstly by varying the dilution rate and secondly by switching the system from continuous culture to batch culture and back again. By gradually increasing the concentration of the toxic constituents, the tolerance of the population may be improved to biodegrade both the readily degradable components and also the more toxic components of the effluent. The procedure may be continued until the population is capable of biodegrading the synthetic waste constituents at a concentration comparable or greater than

3

those likely to be encountered in the actual waste effluents.

The adapted mixed microbial population thus developed may be harvested and stored under glycerol (30% v/v) at -18°C or by other conventional methods of maintaining microorganisms and used as the inocula to biodegrade the actual waste effluents. The initial study on the waste biodegradation may be performed using a conventional single pass continuous culture system. Biodegradation of the waste effluents is conveniently monitored by removal of chemical oxygen demand (COD). Once successful biodegradation has been achieved in a simple continuous culture system, the microbial population may be used in a pilot scale activated sludge system or other effluent treatment biosystems prior to scale-up.

The present invention will now be exemplified by illustrative reference to the microbial degradation of aqueous waste effluents produced during the manufacture of acrylonitrile.

One conventional route for the chemical manufacture of acrylonitrile is via the direct oxidation of propylene in the presence of ammonia, a process known as ammoxidation. A consequence of manufacturing processes for acrylonitrile production is the production of highly toxic aqueous waste effluents which contain a complex mixture of organic nitriles, amides, acids and other compounds. The waste effluents cannot be released into the environment due to toxicity and high COD value. They are usually disposed of by chemical means, by deep well injection or by other conventional methods.

A typical analysis of one such waste effluent is given in Table 1 below. As will be appreciated by those skilled in the art, such waste streams may vary considerably in terms of flow and composition, for example.

## Table 1

### (a) Gross composition

mg/l

| Component | WWCB | SCB |
|---|---|---|
| ammonia nitrogen | 22,000 | 160 |
| Kjeldhal nitrogen | 46,600 | 180 |
| $BOD_5$ | 9,000 | 3,000 |
| total organic carbon | 11,000 | 4,300 |
| COD (chromate) | 22,000 | 10,000 |
| chloride | 100 | 3 |
| dissolved solids | 98,100 | 3,880 |
| suspended solids | 140 | 7 |
| total solids | 98,200 | |
| volatile Dis. (solvd. solids) | 96,200 | 3,200 |
| volatile suspended solids | 97 | 6 |
| sulphate ($SO_4$) | 76,000 | 58 |
| cyanide total | 1,000 | 450 |
| pH | 4.7 | 5.8 |
| flow (kg $h^{-1}$) | 27,000 | 29,000 |
| flow (litres $min^{-1}$) | 900 | 573 |

### (b) Major organic components

| | WWCB | | SCB | |
|---|---|---|---|---|
| | Avg. | Range | Avg. | Range |
| Acetaldehyde | 28 | 16-39 | 5 | 2-9 |
| Acetone | - | - | - | - |
| Acrolein | 52 | 34-91 | 36 | 29-49 |
| Acetic Acid | 1310 | 845-1796 | 2323 | 724-6700 |
| Acrylic Acid | 1827 | 1522-2075 | 786 | 319-1860 |
| Fumaronitrile | 794 | 597-948 | 66 | 32-112 |
| Cyanopyridine | 620 | 488-724 | 65 | 36-108 |
| Acrylamide | 821 | 556-1148 | 49 | 14-121 |
| Maleimide | 1818 | 1596-2292 | 91 | 35-177 |
| Succinonitrile | 231 | 121-414 | 2380 | 1859-3005 |
| $NH_3$ | 1.98% | 1.60-2.46% | 547 | 535-564 |
| Acidity | .26% | .204-.387% | .01 | 0-.05% |
| HCN | 445 | 350-640 | 13 | 7-30 |
| pH | 4.92 | 4.89-4.97 | 5.07 | 5.03-5.10 |
| Density | 1.05 | 1.03-1.07 | | .987-.995 |
| $(NH_4)_2SO_4$ | 7.05% | 5.9-9.1% | | - |
| Tar | .31% | .24%-.38% | .44 | .35-.61% |

(The major organic components are expressed in ppm (mg/l) and other components in percent (w/v).)

There are two major effluents, the wastewater column bottoms (WWCB) and the stripper column bottoms (SCB).

The microbial metabolism of both aliphatic and aromatic nitriles has been investigated in detail over the past fifteen years. Aliphatic nitriles are catabolised in two stages, via conversion to the corresponding amide and then to the acid plus ammonia by a nitrile hydratase and an amidase, respectively. Benzonitrile and related aromatic nitriles and an heterocyclic nitrile are converted directly to the corresponding acids and

EP 0 274 856 B1

ammonia via a nitrilase; little free amide is formed and for production of the acid there is no requirement for amidase activity.

The microorganism Nocardia rhodochrous LL100-21 has been shown to grow on a range of aliphatic nitriles and amides as well as benzonitrile and benzamide as carbon and/or nitrogen sources. Brevibacterium R312 utilises aliphatic nitriles by a nitrile hydratase enzyme that has a broad substrate specificity and a non-specific amidase.

Acrylonitrile has been demonstrated to act only as a nitrogen source for growth of Nocardia rhodochrous LL100-21 and Brevibacterium R312, while an Arthrobacter species has been isolated that could utilise acrylonitrile as sole source of carbon and nitrogen. Acrylamide, a known neurotoxin, may also be utilised by these microorganisms, and has been shown to be biodegraded in soils.

Acrylic acid is a potent inhibitor of $\beta$-oxidation. Certain microorganisms may, however, utilize acrylic acid, and it has been found as an intermediate in the fermentation of propionate by Clostridia propionicum.

3-Cyanopyridine has been reported to act as a substrate for the production of nicotinamide by Brevibacterium R312. It has also been shown to be a substrate for the nitrilase enzyme of Nocardia sp. N.C.I.B. 11215 when grown on benzonitrile. The further metabolism of the acid product of 3-cyanopyridine, nicotinic acid, has been elucidated in a pseudomonas species and subsequently the enzymology of the pathway has been studied. There has been described an Aeromonas sp. that biodegrades succinonitrile as a source of nitrogen, simultaneously hydrolysing the two terminal nitrile groups. There has also been described succinonitrile metabolism by the fungus Fusarium solani.

Cyanide is highly toxic to living organisms. However, a range of microorganisms have previously been isolated that are able to degrade or utilise cyanide. Several fungi that are pathogenic to cyanide-producing plants, as well as other fungi, are known to be able to degrade cyanide to non-toxic formamide by the enzyme cyanide hydratase. Bacteria, especially Pseudomonas fluorscens species, have previously been isolated that utilise cyanide as the source of nitrogen for growth by its conversion to ammonia by the cyanide oxygenase enzyme system. Other microbial cyanide-utilising enzyme systems are known to occur.

Fumaronitrile is an unsaturated dinitrile which exhibits broad spectrum toxicity. The use of fumaronitrile as a bacteriocide is known. Fumaronitrile was added to an emulsion containing a copolymer of ethyl acrylate, methyl acrylate and itaconic acid and a non-ionic emulsifier at a concentration of 0.05% (w/v). The emulsion was inoculated with a bacterial flora and incubated at 25°C, and no bacterial growth was observed over a period of six months. Fumaronitrile is also known as an antiseptic for metal cutting fluids. Fumaronitrile (50ppm) was added to an oil-water mixture containing $4.7 \times 10^4$ bacteria ml$^{-1}$. After seven days storage at 30°C, no viable organisms were found in the mixture.

Acrolein is a highly volatile aldehyde that has been used widely as a herbicide to control submerged aquatic weeds in irrigation channels. The loss of acrolein from water by volatilization has been studied and the reaction of acrolein with water has been reviewed. The primary reaction is reversible hydrolysis to $\beta$-hydroxypropionaldehyde which is less volatile than acrolein.

It may therefore be seen that some of the major toxic components of the effluents from acrylonitrile manufacture might be expected to be readily biodegraded (e.g. acrylonitrile, acrylamide, acrylic acid, cyanopyridine and succinonitrile), but various other components could be much more recalcitrant (e.g. maleimide, fumaronitrile and acrolein). Furthermore, the presence of a range of these compounds will exert a profound inhibitory effect on the development of activated sludge systems and, indeed, such acclimated systems have not been obtained by the gradual exposure to the acrylonitrile effluent.

The present invention enables the development of a microbial system to biodegrade the acrylonitrile waste effluents. As a first step, microorganisms were sought and found which could biodegrade or biotransform the major individual components of the waste effluents. Thereafter, a microbial population has been developed, using a mixture of the isolates, that is competent to degrade the acrylonitrile wastes in an activated sludge system.

Microorganisms were obtained from readily available sources that could biodegrade or biotransform each of the major components of the acrylonitrile waste effluent, viz, succinonitrile, 3-cyanopyridine, acrylic acid, acrylamide, acrolein, acrylonitrile, fumaronitrile and maleimide. By classical enrichment, organisms were obtained that could utilize succinonitrile, 3-cyanopyridine, acrylamide, acrylonitrile and maleimide each as sole source of carbon and nitrogen and acrylic acid as sole carbon source, mineralising these compounds to carbon dioxide, ammonia and biomass. See Table 2 below:

6

Table 2

| Pure Culture Isolates obtained from all enrichments | | | |
|---|---|---|---|
| Substrate | Sole source carbon and nitrogen | Sole source of carbon | Code number of isolates |
| Succinonitrile | + (3) | NT | SN-1, SN-5 SN-8 |
| Fumaronitrile | - | - | - |
| Acrylonitrile | + (1) | NT | AN-2 |
| Acrylamide | + (3) | NT | AM-1(a), AM-2 AM-C |
| Acrolein | NT | - | |
| Acrylic Acid | NT | + (2) | AA-1, AA-2 |
| 3-Cyanopyridine | + (2) | NT | CYP-A, CYP-B |
| Maleimide | + (1) | NT | MAL-B |
| (NT = not tested) | | | |

Each of the substrates was mineralised in approximately 30 hours in batch culture when added at a starting concentration of 20 or 25mM, except for the slow growing organism capable of mineralising maleimide. Certain relevant growth characteristics of the microorganisms are given in Table 3 below:

Table 3

| Substrate | Conc. of substrate (mM) | Organism | Doubling time $T_D$(h) | Final $NH_3$ conc.(mM) |
|---|---|---|---|---|
| Succinonitrile | 25 | SN-1 | 8.5 | 39.5 |
| Succinonitrile | 25 | SN-5 | 6.5 | 39.5 |
| Succinonitrile | 25 | SN-8 | 3.5 | 46.5 |
| Acrylamide | 25 | AM-C | 5 | 11.5 |
| Acrylamide | 25 | AM-2 | 4.5 | 19 |
| Acrylamide | 25 | AM-1(a) | 4.5 | 17 |
| 3-Cyanopyridine | 25 | CYP-A | 5.5 | 47.5 |
| 3-Cyanopyridine | 25 | CYP-B | 5.5 | 47.5 |
| Acrylic acid | 25 | AA-1 | 17 | NA |
| Acrylic acid | 25 | AA-2 | 15 | NA |
| Acrylonitrile | 10 | AN-2 | 2.5 | 8.25 |
| Maleimide | 10 | MAL-B | 46.8 | 8 |
| (N.A. = Not applicable) | | | | |

Isolates were not obtained that were capable of biodegrading fumaronitrile and acrolein. The isolates capable of growth on the various nitrile compounds were tested for the ability thereof to biotransform fumaronitrile as nitrile hydratase enzymes have in certain microorganisms previously been demonstrated to be relatively non-specific. Organism CYP-A isolated for its ability to utilize 3-cyanopyridine as sole source of carbon and nitrogen was demonstrated rapidly to biotransform fumaronitrile to 3-cyanopropenoic acid. This biotransformation product was readily mineralised by two other isolates obtained by enrichment. Thus, a system for the biodegradation of fumaronitrile was obtained via biotransformation by one organism to 3-cyanopropenoic acid and biodegradation of this compound by other microorganisms to carbon dioxide, ammonia and biomass. Enrichment on analogues of the other components maleimide and acrolein yielded microorganisms capable of biotransforming these compounds to readily biodegraded components, maleimide to maleamic acid and acrolein to acrylic acid.

A simplified synthetic waste effluent containing 3-cyanopyridine, succinonitrile, acrylamide and acrylic acid (all 5mM) was biodegraded in a conventional continuous culture system using a defined mixture of all the microorganisms obtained from the enrichments described above, together with available cyanide-utilising Pseudomonas fluorescens species. Once a stable mixed culture was obtained on this synthetic waste, the concentration of the more toxic components fumaronitrile and maleimide was gradually increased. The mixed microbial population was adapted by varying dilution rates and switching the system between continuous and batch culture. By the adaptation procedures, the mixed microbial population was

EP 0 274 856 B1

adapted to biodegrade a synthetic waste containing succinonitrile, 3-cyanopyridine, acrylamide, acrylic acid, fumaronitrile and maleimide (all at 5mM) acrylonitrile and cyanide (1mM) and acrolein (0.5mM) converting all of these completely to carbon dioxide, ammonia and biomass.

The adapted mixed microbial population was used to biodegrade the effluents produced during manufacture of acrylonitrile. The two major effluents were WWCB and SCB as described earlier. The SCB effluent was readily degraded, over 75% of the waste, as determined by COD, was rapidly mineralised. The WWCB effluent proved to be more toxic, however, when diluted 1 part in 10 the mixed population degraded 75-80% of the COD of this effluent. The two effluents may be treated by a distillation step prior to biodegradation. The SCB condensate and the WWCB condensate were both degraded by the microbial population (the SCB condensate over 90% removal of COD, the WWCB condensate over 80-85% removal of COD.)

The manufacturing plant was adapted so that the WWCB condensate was altered to give only one effluent, termed the mixed condensate. Over 75% removal of COD of this effluent was achieved by the mixed microbial population.

Residual COD was almost entirely due to non-toxic oligometric and polymeric material. All toxic compounds were below the levels that could be detected by HPLC and GLC and other conventional analytical techniques.

As will be appreciated, this exemplification relates to a laboratory-scale model of a system in accordance with the present invention and there is every expectation that further improvements may be obtained when such a system is optimised in a given industrial situation. For example, the samples utilised for this study were unavoidably stored prior to testing thus increasing the possibility of polymerisation. In situ samples are known to have lower levels of oligomeric or polymeric material, particularly the condensates.

The biodegradative process may either be by continuous culture or by an activated sludge system or any other biological treatment system. See Table 4 below:

Table 4

| Biodegradation of AN Wastes by the Adapted Mixed Microbial Population | |
|---|---|
| Effluent | Percentage biodegradation (COD removal) |
| Stripper column bottoms (SCB) | >75% |
| Wastewater column bottoms (WWCB)(1 in 10 dilution) | 75-80% |
| SCB condensate | >90% |
| WWCB condensate | 80-85% |
| Mixed condensate (continuous culture) | 75% |
| Mixed condensate (activated sludge system) | 80% |

The bacterial isolates referred to herein are listed in Table 5 below:

8

Table 5

| Organism Code | Isolation Substrate | Identification |
|---|---|---|
| SN-1 | Succinonitrile (C + N) | Alcaligenes sp. |
| SN-5 | Succinonitrile (C + N) | NI |
| SN-8 | Succinonitrile (C + N) | Pseudomonas sp. |
| AM-1(a) | Acrylamide (C + N) | Alcaligenes sp. |
| AM-2 | Acrylamide (C + N) | NI |
| AM-C | Acrylamide (C + N) | NI |
| CYP-A | 3-Cyanopyridine (C + N) | Pseudomonas sp. |
| CYP-B | 3-Cyanopyridine (C + N) | Flavobacterium sp. |
| AN-2 | Acrylonitrile (C + N) | Acinetobacter sp. |
| AA-1 | Acrylic Acid (C) | NI |
| AA-2 | Acrylic Acid (C) | NI |
| MAL-B | Maleimide (C + N) | Bacillus megaterium |

NI - not identified, C - carbon source only, N - nitrogen source only, C + N - sole source of carbon and nitrogen.

The present invention relates in one preferred embodiment to the isolation of bacteria able to biodegrade and mineralise each of the toxic components found in the complex waste effluents from the manufacture of acrylonitrile. These isolates may be used in mixed cultures to biodegrade synthetic mixtures of the major components of the acrylonitrile waste effluents or in the actual waste effluents. The development of stable mixed cultures required the evolution of a population that is capable of cometabolising certain compounds in the mixture which no individual organisms are capable of utilizing as growth substrates.

Further according to the present invention, the microorganisms have been utilized in a complex mixed culture to biodegrade mixtures of the components of the AN waste effluents in synthetic and actual industrial wastes. The mixed microbial population was able readily to biodegrade succinonitrile, acrylamide, acrylic acid, 3-cyanopyridine and acrylonitrile, but the more toxic and recalcitrant compounds maleimide, cyanide, acrolein and fumaronitrile were not readily utilized. However, inter alia, by using a combination of differing dilution rates, switching the system between batch and continuous and gradually increasing the concentration of the two recalcitrants, maleimide and fumaronitrile, the mixed microbial population has been adapted to give a stable population able to biodegrade all the major components of the AN waste in a synthetic mixture, mineralising them to carbon dioxide, ammonia and biomass.

The mixed microbial population after being adapted to mineralise the synthetic effluent can biodegrade the actual AN wastes (SCB and WWCB) and condensates of these wastes removing all detectable toxic components. The end products of mineralisation of the toxic components are carbon dioxide ammonia and biomass. The residual COD after biodegradation is composed of non-toxic polymeric material.

The present approach to develop a mixed culture system capable of biodegrading each of the components of a waste effluent may have many applications in the biotechnological treatment of toxic industrial waste effluents.

Studies to date of the biodegradation of xenobiotics have generally been of two types. Firstly, the degradation of a single compound by pure or mixed cultures of microorganisms obtained either by classical enrichment or by continuous enrichment. Secondly, mixtures of xenobiotics, usually industrial wastes, have been biodegraded by the gradual adaptation of activated sludge systems to tolerate previously toxic levels of various compounds. An example of the latter is the use of activated sludge systems to treat coke-oven liquors.

Using a defined mixed culture of bacteria obtained by enrichment for individual bacteria on each of the major individual components of a waste effluent, followed by combining the isolates to degrade the complex effluent is a novel, advantageous approach to microbial degradation of toxic waste effluents. Furthermore, the ability of the mixed population to tolerate increased concentrations of the more toxic constituents in continuous culture and activated sludge indicates that this approach may be used to develop degradative communities for the detoxification of industrial wastes previously thought to be recalcitrant to microbial degradation.

9

**Claims**

1. A process for the microbial degradation of waste characterised in that it comprises: determining the significant constituent(s) of a waste, providing one or more microorganisms able at least partially to degrade and/or biotransform and/or mineralise each determined constituent of the waste, optionally providing one or more other microorganisms capable of furthering mineralisation of biotransformation or partial degradation product(s), growing one or more mixed cultures of at least some of the microorganisms on a synthetic mixture of at least some of the determined constituents of the waste and utilising the adapted population of microorganisms substantially to mineralise the actual waste.

2. A process as claimed in claim 1 wherein the mixed culture(s) is/are further adapted to optimise mineralisation of the synthetic waste.

3. A process as claimed in claim 1 or claim 2 wherein the mixed culture(s) is/are sequentially adapted by addition of the determined constituent(s) and/or by varying the dilution rate and/or by switching between batch and continuous culture.

4. A process as claimed in any of claims 1 to 3 wherein the microorganisms are provided by identification and isolation.

5. A process as claimed in any of claims 1 to 4 wherein the adapted population is optimised substantially to mineralise the actual waste.

6. A process as claimed in any of claims 1 to 5 wherein the adapted population of microorganisms is utilised substantially to mineralise the actual waste in an activated sludge system.

7. A process as claimed in any of claims 1 to 6 wherein the waste is from acrylonitrile production.

**Revendications**

1. Procédé de dégradation microbienne des déchets, caractérisé par le fait qu'il comporte le fait de déterminer le (ou les) constituant (s) significatif (s) des déchets, de fournir un ou plusieurs microorganismes capables, au moins partiellement, de dégrader et/ou de biotransformer et/ou de minéraliser chaque constituant déterminé des déchets, en option defournir un (ou plusieurs) autre (s) microorganisme (s) capable (s) de poursuivre la minéralisation ou la biotransformation ou la dégradation partielle du (ou des) produit (s), de faire croître une ou plusieurs cultures mixtes d'au moins certains des micro-organismes sur un mélange synthétique d'au moins certains des constituants déterminés des déchets et d'utiliser la population adaptée de micro-organismes pour substantiellement minéraliser les déchets réels.

2. Procédé selon la revendication 1, dans lequel on adapte en outre la (ou les) culture (s) mixte (s), pour optimiser la minéralisation des déchets synthétiques.

3. Procédé selon la revendication 1 ou la revendication 2, dans lequel on adapte séquentiellement la (ou les) culture (s) mixte (s), en ajoutant le (s) constituant (s) déterminé (s) et/ou en faisant varier le taux de dilution et/ou en passant alternativement d'une culture par lots à une culture continue.

4. Procédé selon l'une des revendications 1 à 3, dans lequel les micro-organismes sont fournis par identification et isolation.

5. Procédé selon l'une des revendications 1 à 4, dans lequel on optimise la population adaptée pour minéraliser substantiellement les déchets réels.

6. Procédé selon l'une des revendications 1 à 5, dans lequel on utilise la population adaptée de micro-organismes pour substantiellement minéraliser les déchets réels dans un système de boue activée.

7. Procédé selon l'une des revendications 1 à 6, dans lequel les déchets proviennent de la production d'acrylonitrile.

**Patentansprüche**

1. Verfahren zum mikrobiellen Abbau von Abfall, **dadurch gekennzeichnet,** daß man den signifikanten Bestandteil bzw. die signifikanten Bestandteile eines Abfalls bestimmt, einen oder mehrere Mikroorganismen bereitstellt, die jeden bestimmten Bestandteil des Abfalls mindestens teilweise abbauen und/oder biotransformieren und/oder mineralisieren können, ggf. einen oder mehrere weitere Mikroorganismen bereitstellt, die die Mineralisation des bzw. der Biotransformations- oder Teilabbauprodukts (Produkte) fördern können, eine oder mehrere Mischkulturen aus mindestens einigen der Mikroorganismen aus einem synthetischen Gemisch auf mindestens einigen der bestimmten Bestandteile des Abfalls züchtet, und die angepaßte Mikroorganismenpopulation im wesentlichen zur Mineralisierung des tatsächlichen Abfalls verwendet.

2. Verfahren nach Anspruch 1, **dadurch gekennzeichnet,** daß die Mischkultur bzw. die Mischkulturen weiter angepaßt ist bzw. sind, um die Mineralisation des synthetischen Abfalls zu optimieren.

3. Verfahren nach Anspruch 1 oder 2, **dadurch gekennzeichnet,** daß die Mischkultur bzw. die Mischkulturen schrittweise durch Zugabe des bestimmten Bestandteils bzw. der bestimmten Bestandteile und/oder durch Variieren der Verdünnungzrate und/oder durch Umschalten zwischen chargenweiser und kontinuierlicher Kultur angepaßt wird bzw. werden.

4. Verfahren nach einem der Ansrpüche 1 bis 3, **dadurch gekennzeichnet,** daß die Mikroorganismen durch Identifikation und Isolierung bereitgestellt werden.

5. Verfahren nach einem der Ansprüche 1 bis 4, **dadurch gekennzeichnet,** daß die angepaßte Population im wesentlichen zur Mineralisierung des tatsächlichen Abfalls optimiert wird.

6. Verfahren nach einem der Ansprüche 1 bis 5, **dadurch gekennzeichnet,** daß die angepaßte Mikroorganismenpopulation im wesentlichen zur Mineralisation des tatsächlichen Abfalls in einem Belebtschlammsystem verwendet wird.

7. Verfahren nach einem der Ansprüche 1 bis 6, **dadurch gekennzeichnet,** daß der Abfall aus der Acrylonitrilherstellung stammt.